# EUROPÄISCHE PATENTANMELDUNG

(11) **EP 2 732 804 A2**
(43) Veröffentlichungstag der Anmeldung: **21.05.2014**
(21) Anmeldenummer: 13192063.9
(22) Anmeldetag: 08.11.2013
(51) Int. Cl.: A61K 8/34, A61Q 19/00, A61K 8/81, A61K 8/04

(54) **Hydrodispersion mit verbesserter Leistung**

(30) Priorität: 20.11.2012 DE 102012221224
(71) Anmelder: Beiersdorf AG, 20253 Hamburg (DE)
(72) Erfinder: Ratschow, Cecile, 22761 Hamburg (DE); Meyer, Christiane, 20357 Hamburg (DE); Schulz, Jens, 25462 Rellingen (DE); von der Fecht, Stephanie, 22880 Wedel (DE); Schlüter, Axel, 25462 Rellingen (DE)

(57) **Zusammenfassung**

Die Erfindung umfasst Hydrodispersionen enthaltend Ethylhexylglycerin und/oder Caprylylglycol, die eine verbesserte Verdickung hervorrufen.

## Beschreibung

Die Erfindung umfasst Hydrodispersionen enthaltend Ethylhexylglycerin und/oder Caprylylglycol, die eine verbesserte Verdickung und einfachere Konservierung hervorrufen.

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Aber auch emulgatorarme bzw. -freie Zubereitungen auf Basis sogenannter Hydrodispersionen sind bekannt. Hydrodispersionen stellen Dispersionen einer flüssigen, halbfesten oder festen inneren (diskontinuierlichen) Lipidphase in einer äußeren wässrigen (kontinuierlichen) Phase dar. Hydrodispersionen sind - wie auch Emulsionen, die sich durch eine ähnliche Phasenanordnung auszeichnen - metastabile Systeme und daher geneigt, in einen Zustand zweier in sich zusammenhängender diskreter Phasen überzugehen. In einer klassischen O/W-Emulsion verhindert die Wahl eines geeigneten Emulgators die Phasentrennung. Im Gegensatz zur klassischen Emulsionen enthalten Hydrodispersionen aber nur sehr geringe Mengen an Emulgatoren (bis zu 2 Gew.-%) bzw. können sogar gänzlich frei von Emulgatoren sein.

Bei Hydrodispersionen, welche aus einer flüssigen Lipidphase in einer äußeren wässrigen Phase bestehen, wird die Stabilität des Mehrphasensystems üblicherweise dadurch gewährleistet, dass in der wässrigen Phase mit Hilfe eines Gelbildners bzw. Verdickungsmittels ein Gelgerüst aufgebaut wird, in welchem die Lipidtröpfchen stabil suspendiert sind.

Als Verdickungsmittel sind Homopolymere oder Copolymere von Acrylsäure und/oder Acrylamid und deren Derivaten bekannt. Formulierungen auf Polyacrylatbasis zeigen jedoch häufig keine nachhaltigen Pflegeeffekte. Gel-Formulierungen sind außerdem salzempfindlich, so dass sie beim Auftragen auf die Haut durch die Präsenz von Salzen häufig brechen.

Des Weiteren fühlen sich Hydrodispersionen im Vergleich zu Emulsionen häufig klebrig an.

Nachteil der Hydrodispersionen des Standes der Technik ist ferner, dass diese bei einem höheren Gehalt an Lipiden (größer 4 Gew.-%) zur Phasentrennung neigen, die insbesondere bei höheren Temperaturen (≥ 40 °C) auftreten kann.

In der WO 2005097057 A1 werden Gelformulierungen offenbart, die polymere Gelbildner, Wasser, Öle sowie mindestens ein Wachs, das einen Schmelzpunkt von 30°C und mehr besitzt, umfassen. Die Formulierungen sind frei von kationischen oder anionischen Emulgatoren oder Tensiden.

In der WO 2005097055 A1 werden Gelformulierungen beschrieben, die polymere Gelbildner, Wasser, eine bei 25°C flüssige Ölphase und mindestens eine Wachskomponente mit einem Schmelzpunkt von wenigstens 30°C ausgewählt aus der Gruppe der Pentaerythritester, der Dipentaerythritester und/oder der Tripentaerythritester, umfassen.

Die sensorischen Eigenschaften dieser Gelformulierungen stellen sich jedoch als nicht so attraktiv dar. Der Rückstand der Formulierung auf der Haut fühlte sich sehr wachsig an.

Wünschenswert ist es daher, Hydrodispersionen bereit zu stellen, die sensorisch verbesserte Eigenschaften aufweisen und insbesondere deren Rückstand weniger wachsig erscheint.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Eine für den Verbraucher sehr wesentliche, dabei aber nur schwer quantitativ messbare Eigenschaft kosmetischer Produkte ist ihre Textur. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.
- *Visuelle Eindrücke:* alle mit dem Auge wahrnehmbaren Merkmale (Farbe, Form, Struktur).
- *Olfaktorische Eindrücke:* alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- *Haptische Eindrücke:* alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

Aufgabe der vorliegenden Erfindung ist es kosmetische oder dermatologische Hydrodispersionen bereit zu stellen, die, gegebenenfalls auch Wachse umfassend, ein angenehmes, sensorisch leichtes Profil und gute Pflegeeffekte aufweisen und bei Applikation auf der Haut nicht brechen.

Verdickungsmittel oder Gelbildner führen in Zubereitungen, wie Hydrodispersionen, zu unvorteilhaftem Abrieb, wenn die auf die Haut aufgetragene Hydrogelschicht leicht und in unschöner Weise ("Röllchenbildung") abgetragen wird.

Es ist daher eine Aufgabe der vorliegenden Erfindung den Anteil an Verdickungsmitteln in kosmetischen Hydrodispersionen zu vermindern ohne Einbußen hinsichtlich Konsistenz und Stabilität hinnehmen zu müssen.

Überraschenderweise wurde nun herausgefunden, dass die eigentlich nur als Konservierungsmittelhelfer bekannten Rohstoffe Ethylhexylglycerin und/oder Caprylylglycol in Hydrodispersionen eine unerwartete Verdickung hervorrufen. Der Anteil an Verdickungsmitteln kann somit verringert werden.

Die Erfindung umfasst daher Hydrodispersionen umfassend Wasser, ein oder mehrere Verdickungsmittel und Ethylhexylglycerin und/oder Caprylylglycol, die eine verbesserte Verdickung hervorrufen.

Die mikrobielle Stabilität derartiger kosmetischer oder dermatologischer Hydrodispersionen wird bislang durch den Zusatz an Konservierungsmitteln gelöst.

Bekannte Konservierungsmittel sind die als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben. Des Weiteren ist Phenoxyethanol (Phenoxetol^{®}),Piroctone Olamine, Ethylenglycolmonophenylether und Phenylglycol zur Konservierung von kosmetischen Mitteln bekannt.

Nachteile von Konservierungsmitteln sind, dass sie Hautallergien hervorrufen können und sie Bakterien (Zellen) töten, dadurch aber auch die Haut schädigen können. Durch Konservierungsmittel oder einem Zuviel an Konservierungsmitteln können Hautunverträglichkeiten auftreten, insbesondere bei Babys und Kleinkindern, die dünnerere Haut haben sowie bei Personen mit empfindlicher kranker Haut.

Kosmetische Zubereitungen müssen jedoch langzeitstabil gegen mikrobielle Kontamination formuliert werden und trotzdem eine sehr gute Hautverträglichkeit aufweisen.

Des Weiteren ist ein einfacher Austausch oder das Vermeiden von Konservierungsstoffen wie Parabene und/oder Phenoxyethanol ohne Einbußen hinsichtlich der Stabilität und Anwendungseigenschaft nicht ohne weiteres möglich.

Wünschenswert ist es daher, die Menge an Konservierungsmitteln und Konservierungsmittelhelfer in kosmetischen oder dermatologischen Zubereitungen weitestmöglich zu reduzieren oder ganz auf sie zu verzichten, ohne jedoch Einbußen in der mikrobiologischen Stabilität hinnehmen zu müssen.

Durch den Zusatz der erfindungsgemäßen Ethylhexylglycerin und/oder Caprylylglycol kann eine deutlich verbesserte mikrobiologische Stabilität erreicht werden ohne die Menge an Konservierungsmitteln erhöhen zu müssen bzw. es kann ganz auf sie verzichtet werden.

Bevorzugt umfassen die erfindungsgemäßen Zubereitungen daher neben Ethylhexylglycerin und/oder Caprylylglycol keine weiteren Konservierungsmittel und Konserviewrungsmittelhelfer, insbesondere keine Parabene, Formaldehydabspalter, organohalogenische Stoffe, Ethanol und/oder Phenoxyethanol.

Die Zubereitungen sind zudem vorteilhaft frei von Polyethylenglykolen und/oder Polyethylenglykolderivaten.

Der Anteil an zusätzlichen Konservierungsmittel, - helfern und/oder PEGs liegt daher unter 1 Gew.%, insbesondere 0 Gew.%, bezogen auf die Gesamtmasse der Zubereitung.

Die erfindungsgemäße Hydrodispersion umfasst neben Wasser, ein oder mehrere Verdickungsmittel, gegebenenfalls ein oder mehrere Wachse und gegebenenfalls ein oder mehrerer Öle sowie Ethylhexylglycerin und/oder Caprylylglycol, insbesondere beide.

Ein weiterer Vorteil ist, dass die erfindungsgemäßen Zubereitungen aufgrund verringerter Anteile an Verdickungsmitteln und/oder Konservierungsmitteln kostengünstiger herzustellen sind als vergleichbare Hydrodipersionszubereitungen des Standes der Technik.

Es sind weniger Verdickungsmittel erforderlich um die gleiche Konsistenz zu bekommen. Die Elektrolytempfindlichkeit der erfindungsgemäßen Zubereitungen wird ebenfalls reduziert.

Außerdem brauchen die erfindunsgemäßen Zubereitungen keine zusätzliche Konservierungsmittel.

Überraschend zeigt sich, dass hinsichtlich Stabilität, Verdickungsleistung als auch Konservierungsleistung dabei keinerlei Einbußen zu beobachten sind.

Es wurden Vergleichsversuche zur Stabilität und Verdickungsleistung durchgeführt.

Die Viskosität der Hydrodispersionen A, B und C (siehe nachfolgende Tabelle) wurden mit der Rheomat 123 der Firma proRheo, (Spindel 1 bei T=25°C in einem 125 ml-Schraubglas) gemessen.

**Tabelle 1: Vergleichsuntersuchung Stabilität und Verdickungsleistung**

| | A | B | C |
|---|---|---|---|
| Piroctone Olamine | 0,05 | 0 | 0 |
| Phenoxyethanol | 0,6 | 0 | 0 |
| Caprylyl Glycol | 0 | 0 | 0,5 |
| Ethylhexylglycerin | 0 | 0,5 | 0 |
| Cetyl Palmitate | 3 | 3 | 3 |
| Butyrospermum Parkii Butter | 3 | 3 | 3 |
| C12-15 Alkyl Benzoate | 4 | 4 | 4 |
| Shorea Stenoptera Seed Butter | 3 | 3 | 3 |
| Parfum | 0,2 | 0,2 | 0,2 |
| Glycerin | 10 | 10 | 10 |
| Aqua + Sodium Hydroxide | 1,09 | 1,09 | 1,09 |
| Glycerin + Glyceryl Glucoside | 0,1 | 0,1 | 0,1 |
| Sodium Polyacrylate | 1,5 | 1,5 | 1,5 |
| Aqua | 66,21 | 66,36 | 66,36 |
| Aqua + Trisodium EDTA | 1 | 1 | 1 |
| Phenylbenzimidazole Sulfonic Acid | 2,25 | 2,25 | 2,25 |
| Octocrylene | 2 | 2 | 2 |
| Butyl Methoxydibenzoylmethane | 2 | 2 | 2 |

Nach einem Tag der Herstellung wurde die Viskosität bei 25°C gemessen zu
A: 2550 mPa s
B: 4900 mPa s
C: 3500 mPa s

D.h. ohne Veränderung der Verdickungsmittelbestandteile oder deren Anteile (Natriumpolyacryat) wurde eine Zunahme der Konsistenz/Viskosität allein durch den Zusatz an Ethylhexylglycerin bzw. Caprylylglycol erreicht.

Zubereitung A zeigte zudem nach 14 Tagen Lagerung bei 40°C eine Ölabscheidung, die durch den Zusatz an Elektrolyten und UV Filtern herrührt. Durch Zusatz vom Caprylylglycol oder Ethylhexylglycerin ist die Formel stabilisiert und verdickt.

Weiterer Vorteil ist, dass auf die Konservierungsmittel - wie beispielsweise Phenoxyethanol und Piroctone Olamine - verzichtet werden kann ohne eine Verschlechterung der mikrobiologischen Stabilität zu erzielen.

Es wurden Vergleichsversuche zur mikrobiologischen Stabilität durchgeführt wie nachfolgende Tabelle zeigt.

**Tabelle 2: Vergleichsuntersuchung mikrobiologische Stabilität**

| **INCI** | **A** | **B** | **C** |
|---|---|---|---|
| Phenoxyethanol | 0,6 | 0 | 0 |
| Piroctone Olamine | 0,05 | 0 | 0 |
| Ethylhexylglycerin | 0 | 0,5 | 0 |
| Caprylyl Glycol | 0 | 0 | 0,5 |
| Glycerin | 9 | 9 | 9 |
| Candelilla Cera | 0,2 | 0,2 | 0,2 |
| Cetyl Palmitate | 1 | 1 | 1 |
| Butyrospermum Parkii Butter | 4 | 4 | 4 |
| Hydrogenated Vegetable Oil | 0,6 | 0,6 | 0,6 |
| Shorea Stenoptera Seed Butter | 5 | 5 | 5 |
| Olus Oil | 3,2 | 3,2 | 3,2 |
| Sodium Polyacrylate | 1,5 | 1,5 | 1,5 |
| Zea Mays Oil + Cl 40800 + Tocopherol | 0,0004 | 0,0004 | 0,0004 |
| Aqua + Sodium Hydroxide | 0,375 | 0,41 | 0,41 |
| Parfum | 0,2 | 0,2 | 0,2 |
| Aqua | 74,2746 | 74,3896 | 74,3896 |
| Konservierung | | | |
| Bakterien | angemessen | angemessen | angemessen |
| Pilze | angemessen | angemessen | angemessen |
| Hefen | angemessen | angemessen | angemessen |

Auch ohne Konservierungsmittel zeigen die erfindungsgemäßen Zubereitungen G und H eine angemessene, im Vergleich zu der konservierungsmittelhaltigen Zubereitung F, die gleiche Konservierungsleistung.

Kosmetische Zubereitungen werden in Bezug auf ihre Fließfähigkeit unterteilt in flüssige Produkte (Lotionen) und festere Produkte (Cremes). Zur genauen Definition der Fließfähigkeit wird bei den Lotionen die Viskosität und bei den Cremes die Konsistenz gemessen.

In einem weiteren Vergleichstest wurden die folgenden Zubereitungen D und E und deren Konsistenz untersucht. Bei festeren Cremes wird anstelle der Viskosität die Konsistenz der Zubereitung gemessen, wie es in DE 2909087 C2 beschrieben ist.

| INCI | D | E |
|---|---|---|
| Phenoxyethanol | 0,6 | 0 |
| Piroctone Olamine | 0,05 | 0 |
| Ethylhexylglycerin | 0 | 0,5 |
| Sodium Polyacrylate | 1,5 | 0,8 |
| Olus Oil | 3,2 | 3,2 |
| Candelilla Cera | 0,2 | 0,2 |
| Cetyl Palmitate | 1 | 1 |
| Butyrospermum Parkii Butter | 4 | 4 |
| Hydrogenated Vegetable Oil | 0,6 | 0,6 |
| Glycerin | 9 | 9 |
| Aqua + Sodium Hydroxide | 0,41 | 0,2 |
| Shorea Stenoptera Seed Butter | 5 | 5 |
| Aqua | 74,44 | 75,5 |
| | | |
| Konsistenzwert nach 14 Tagen | 20 | 20 |

Auch hier zeigt sich, dass trotz weniger Verdickereinsatz (Natriumpolyacrylat) durch den Einsatz von Ethylhexylglycerin eine Zubereitung mit gleicher Konsistenz hergestellt werden kann.

Durch den Wegfall der Konservierungsmittel und die Reduktion der Verdickungsmittel lassen sich die Kosten der Formulierung verringern, was einen nicht zu unterschätzenden Vorteil darstellt.

Ethylhexyglycerin oder auch 1,2-Propanediol, 3-(2-Ethylhexyloxy) genannt (CAS 70445-33-9) ist als hautpflegender Kosmetikbestandteil bekannt. Auch als Konservierungsmittelhelfer wird Ethylhexylglycerin erwähnt.

Erfindungsgemäß wird auf den Zusatz weiterer Konservierungsmittel oder -helfer verzichtet bzw. deren Anteil begrenzt, vorteilhaft auf einen Anteil von weniger 0,1 Gew.%, bezogen auf die Gesamtmasse der Dispersion.

Ethylhexylglycerin als Konservierungsmittelhelfer ist dann natürlich nicht von der Reduzierung bzw. dem Verzicht umfasst.

Caprylylglykol, auch Octan-1,2-diol bezeichnet (CAS 1117-86-8) ist als geschmeidig machender, feuchthaltender und haarkonditionierender Kosmetikbestandteil bekannt.

Der Anteil an Ethylhexylglycerin und/oder Caprylylglycol beträgt bevorzugt 0,1 bis 1 Gew.%, insbesondere im Bereich von 0,2 bis 0,6 Gew.%, bezogen auf die Gesamtmasse der Zubereitung. Es können erfindungsgemäß auch beide Stoffe enthalten sein.

Deren Einzelanteile liegen dann im oberen Bereich, wobei vorteilhaft die Gesamtmenge den Anteil von 1 Gew.-% nicht überschreitet.

Entgegen den Gelformulierungen des Standes der Technik sind die erfindungsgemäßen Hydrodispersonen darüber hinaus auch sensorisch attraktiv.

Die erfindungsgemäße Dispersion weist eine überraschend reichhaltige Textur bei der Entnahme auf.

Das oder die Verdickungsmittel werden erfindungsgemäß vorteilhaft aus einer oder mehreren der folgenden Gruppen gewählt:
- Homopolymere oder Copolymere von Acrylsäure und/oder Acrylamid und deren Derivaten,
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Xanthan Gum
- organische, abgewandelte Naturstoffe, wie z. B. Carboxymethylcellulose und andere Celluloseether, Hydroxyethyl- und -propylcellulose und mikrokristalline Cellulose oder dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane, AMPS Copolymere wie z.B. Ammonium Acryloyldimethyltaurate/VP Copolymer und Sodium Acrylate/ Acryloyldimethyltaurate/Dimethylacrylamide Crosspolymer, Acrylic Acid/VP Crosspolymer, PVP, Acrylamide/AmmoniumAcrylate Copolymer.

Besonders bevorzugte Verdickungsmittel im Sinne der vorliegenden Erfindung sind Polyacrylate, d. h. Polymere auf Basis von Estern der Acrylsäure *(Polyacrylsäureester)* der allgemeinen Strukturformel 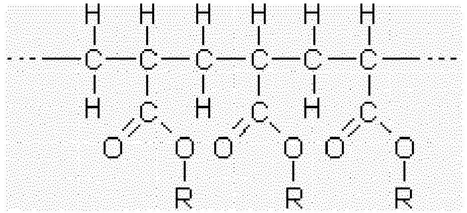 worin R für lineare, verzweigte oder cyclische, ggf. funktionelle Substituenten (z. B. Hydroxy-, Amin- oder Epoxid-Gruppen) enthaltende Alkyl-Reste steht, z. B. für Methyl-, Ethyl-, Isopropyl-, *tert*-Butyl-, Cyclohexyl-, 2-Ethylhexyl-, Dodecyl-, 2-Hydroxyethyl- und 2-Dimethylaminoethyl-.

Ganz besonders bevorzugt sind Polyacrylate mit der INCI-Bezeichnung Sodium Polyacrylate, beispielsweise das unter der Handelsbezeichnung Cosmedia SP bei der Fa. Cognis erhältliche.

Der Anteil an Verdickungsmitteln wird bevorzugt im Bereich von 0,05 bis 5 Gew.%, insbesondere 0,1 bis 2 Gew.% gewählt, bezogen auf die Gesamtmasse der Zubereitung.

Unter dem Begriff Wachs werden üblicherweise alle natürlichen oder künstlich gewonnenen Stoffe und Stoffgemische mit folgenden Eigenschaften verstanden: Sie sind bei Raumtemperatur (20°C) von fester bis brüchig harter Konsistenz, grob bis feinkristallin, durchscheinend bis trüb und schmelzen oberhalb von 20°C ohne Zersetzung. Sie sind schon wenig oberhalb des Schmelzpunktes niedrigviskos und nicht fadenziehend und zeigen eine starke temperaturabhängige Konsistenz und Löslichkeit.

Als Wachse können erfindungsgemäß auch Fette und fettähnlichen Substanzen mit wachsartiger Konsistenz eingesetzt werden. Hierzu gehören u.a. Fette (Triglyceride), Mono und Diglyceride, natürliche und synthetische Wachse, Fett-und Wachsalkohole, Ester von Fettalkoholen und Fettsäuren sowie Fettsäureamide oder beliebige Gemische dieser Substanz.

Besonders bevorzugt werden die Wachse aus der Gruppe der Fette, insbesondere aus
- Shorea Stenoptera Seed Butter
- Hydrogenated Vegetable Oil
- Hydrogenated Coco-Glycerides
- Butyrospermum Parkii Butter
- Theobroma Cacao (Cocoa) Seed Butter
- Mango Butter,
   aus der Gruppe der natürlichen Wachse, insbesondere aus
- Candellila Cera,
   aus der Gruppe der Ester aus Fettsäuren, insbesondere aus
- Methyl Palmitate,
- Cetearyl Behenate,
- Cetyl Palmitate,
   aus der Gruppe der synthetischen Wachse, insbesondere aus
- Synthetic Beeswax,
   sowie aus der Grupper der Fettalkohole, insbesondere aus
- Cetearyl Alcohol
- Cetyl Alcohol
- Stearyl Alcohol
- Behenyl Alcohol,
gewählt.

Der Anteil an einem oder mehreren Wachsen beträgt vorteilhaft bis zu 20 Gew.%, insbesondere 2 bis 15 Gew.%, bezogen auf die Gesamtmasse der Zubereitung.

Bevorzugt sind Hydrodispersion mit einem Anteil von 0,05 - 5 Gew.% wenigstens eines Verdickungsmittels, 2 bis 20 Gew.% eines oder mehrerer Wachse und bis 20 Gew.% eines oder mehrerer Öle, jeweils bezogen auf die Gesamtmasse der Dispersion.

Werden Einschränkungen auf bevorzugte Anteilsbereiche einer oder mehrerer Inhaltsstoffe vorgenommen und/oder Einschränkungen auf bestimmte Inhaltsstoffe so beziehen sich die bevorzugten Anteilsbereiche dann auf die ausgewählten Inhaltsstoffe.

Die erfindungsgemäßen Zubereitungen sind gegenüber den Zubereitungen des Standes der Technik vorteilhaft frei von anionischen und kationischen Emulgatoren und Tensiden.

Sind jedoch aufgrund von Rohstoffverunreinigungen oder sonstigen Einschleppungen anionische oder kationische emulgierende Stoffe oder waschaktive Substanzen in den Zubereitungen enthalten, so gelten diese Zubereitungen erfindungsgemäß immer noch als "frei von" Emulgatoren und Tensiden, sofern deren Anteil nicht über 0,1 Gew.%, bezogen auf die Gesamtmasse der Zubereitung, beträgt.

Verdickungsmittel, die u.U. auch emulgierende Eigenschaften haben können, werden jedoch nicht zu den auszuschließenden Emulgatoren gezählt, da deren Hauptfunktion nicht die eines Emulgators ist.

Die erfindunsgemäßen Hydrodispersion sind bevorzugt als kosmetische oder dermatologische Zubereitung zu formulieren und anzuwenden.

Ethylhexylglycerin und/oder Caprylylglycol lassen sich erfindungsgemäß in Hydrodispersionen, umfassend ein oder mehrere Verdickungsmittel, gegebenenfalls ein oder mehrere Wachse und gegebenenfalls ein oder mehrerer Öle, zur Verdickung der Dispersion verwenden.

Ebenso lassen sich Ethylhexylglycerin und/oder Caprylylglycol in sehr dünnflüssigen Hydrodispersionen bzw. Tränkungsmedien für Tücher, umfassend ein oder mehrere Verdickungsmittel, einarbeiten.

Erstaunlich zeigte sich auch die Verdickungsleistung von Ethylhexylglycerin im Up-scaling von Zubereitungen vom Labor- in den Produktionsmaßstab für Tränkungsmedien von Tüchern für die kosmetische Anwendung. Ohne zusätzliche Verdickungsmittel oder Emulgatoren wird eine Verdickung beim Up-Scaling erzielt, wobei Viskositätswerte erzielt werden im Bereich von 50 mPas bis zu 1000 mPas. Dies ist insbesondere bei der Herstellung von Tränkungsmedien bei der Tücherherstellung vorteilhaft. Dabei wird Ethylhexlglycerin kalt zur Hydrodispersion dazugegeben und eine vorteilhafte Verdickungsleistung von bis zu 10% erreicht. Dadurch wird die Stabilität des Tränkungsmediums insbesondere bei höheren Temperaturen bis zu 45°C optimiert.

Ethylhexylglycerin kann bevorzugt zur Verdickung von Hydrodispersionen im up-scaling Prozess verwendet werden.

Die erfindungsgemäße Hydrodsipersion ist als Tränkungsmedium für Tücher zu verwenden, da die Disperison nur eine Viskosität von mindestens 50mPas bis zu 1000 mPas aufweist. Die erfindungsgemäßen Hydrodispersion kann neben den Wachsen auch andere Öle oder Lipide enthalten, vorteilhaft zu einem Anteil bis zu 20 Gew.%, insbesondere bis zu 10 Gew.%, bezogen auf die Gesamtmasse der Zubereitung.

Als zusätzliche Lipide werden bevorzugt gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Arganöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodecanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (Cetiol OE) und/oder Dicaprylylcarbonat, beispielsweise das unter der Handelsbezeichnung Cetiol CC bei der Fa. Cognis erhältliche.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Die erfindungsgemäßen Zubereitungen können optional einen Gehalt an cyclischen und/oder linearen Silikonölen aufweisen.

Vorteilhaft werden Cyclomethicone, insbesondere Octamethylcyclotetrasiloxan, Cyclomethicone D5 und/oder Cyclomethicone D6 als erfindungsgemäßes Silikonöl eingesetzt. Ein weiteres erfindungsgemäß vorteilhaftes Silikonöl ist Dimethicone (auch: Dimethylpolysiloxan bzw. Polydimethylsiloxan mit der INCI-Bezeichnung Dimethicone).

Aber auch andere Silikonöle sind vorteilhaft im Sinne der vorliegenden Erfindung zu verwenden, beispielsweise Hexamethylcyclotrisiloxan, Poly(methylphenylsiloxan), Phenyltrimethicon, Phenyldimethicon, Hexamethylcyclotrisiloxan, Polydimethylsiloxan, Poly(methylphenylsiloxan), Cetyldimethicone, Behenoxydimethicone.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyethercopolymere wie das Cetyl-Dimethicon-Copolyol oder auch das Lauryl-Methicone-Copolyol.

Besonders vorteilhaft zur Verwirklichung der erfindungsgemäßen Textur wird eine Mischung von cyclischen und linearen Silikonölen, insbesondere von Cyclomethicone und Dimethicone, verwendet. Der Anteil an ein oder mehreren Silikonöle, besonders bevorzugt Cyclomethicone und Dimethicone, wird bevorzug gewählt im Bereich von 0,1 bis 10 Gew.-%, besonders 0,5 bis 8 Gew.-%, jeweils bezogen auf das Gesamtgewicht der Zubereitung.

In einer besonders bevorzugten Ausführungsform enthalten die Zubereitungen im Sinne der vorliegenden Erfindung sogenannte Moisturizer. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Vorteilhaft umfasst die erfindungsgemäße Zubereitung Glyceryl Glucoside, insbesondere zu einem Anteil von 0,001 % bis 8 Gew.%, bezogen auf die Gesamtmasse der Zubereitung.

Vorteilhaft wird die Menge an Moisturizern, eine oder mehrere Verbindungen, aus dem Bereich von 1 bis 20 Gew.-%, bevorzugt von 3 bis 15 Gew.-%, besonders bevorzugt von 5 bis 12 Gew.-% - jeweils bezogen auf das Gesamtgewicht der Zubereitung - gewählt.

Die kosmetische Zusammensetzungen im Sinne der vorliegenden Erfindung können, je nach ihrem Aufbau, beispielsweise verwendet werden als Hautschutzcreme, Reinigungsmilch, Sonnenschutzlotion, Nährcreme, Tages- oder Nachtcreme usw. Besonders bevorzugt im Sinne der vorliegenden Erfindung sind Zubereitungen zur, insbesondere großflächigen Anwendung im Körperpflegebereich.

Vorteilhaft wird die Hydrodsipersion oder Zubereitung als Hautpflegecreme verwendet.

Die Vorteile der erfindungsgemäßen Hydrodispersion sind deren leichte Verteilbarkeit, erfrischender Effekt bei der Applikation und dennoch eine pflegende Wirkung, die insbesondere durch die Wachse hervorgerufen werden.

Die erfindungsgemäße Hydrodisersion ist besonders hautverträglich, da wenig bzw. keine Öle, insbesondere keine spreitende Öle, wenig Emulgatoren und wenig Konservierungsmittel enthalten sind. Eine Anwendung der Hydrodispersion am Auge ist damit problemlos möglich.

Die Zusammensetzungen können auch UV-Filter enthalten gewählt aus der Gruppe der Verbindungen 2-Phenylbenzimidazol-5-sulfonsäure und/oder deren Salze, Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; Diethylamino Hydroxybenzoyl Hexyl Benzoate, 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester;Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer;Dioctylbutylamidotriazon (INCI: Diethylhexyl-Butamidotriazone); 2,4-bis-[5-1 (dimethylpropyl) benzoxazol-2-yl-(4-phenyl)-imino]-6-(2-ethylhexyl)-imino-1,3,5-triazin mit der (CAS Nr.288254-16-0); 4,4',4"-(1,3,5-Triazin-2,4,6-triyltriimino)-tris-benzoesäure-tris(2-ethylhexylester)(auch: 2,4,6-Tris-[anilino-(p-carbo-2'-ethyl-1'-hexyloxy)]-1,3,5-triazin (INCI: Ethylhexyl Triazone);2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine, Butyl Methoxydibenzoylmethane, Octocrylene, sowie Titandioxide und Zinkoxide.

Die kosmetischen oder dermatologischen Zubereitungen gemäß der Erfindung können ferner kosmetische Hilfsstoffe und Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Anti-Aging-Substanzen oder andere übliche Bestandteile einer kosmetischen oder dermatologischen Formulierung wie Polyole, Polymere, Schaumstabilisatoren, organische Lösungsmittel oder Silikonderivate, sofern der Zusatz die geforderten Eigenschaften hinsichtlich der Sensorik nicht beeinträchtigt und die geforderten Eigenschaften der Verdickung und Konservierung nicht verhindert wird.

Folgende Wirkstoffe können z.B. enthalten sein, gewählt aus der Gruppe der Verbindungen Glycyrrhetinsäure, Harnstoff, Arctiin, alpha-Liponsäure, Folsäure, Phytoen, D-Biotin, Coenzym Q10, alpha-Glucosylrutin, Tocopheryl Acetate, Carnitin,Carnosin, Coffein, natürliche und/oder synthetische Isoflavonoide, Kreatin,Kreatinin, Taurin, Magnolia, ß-Alanin und/oder Licochalcon A.

Der Wasseranteil der Hydrodisperison wird vorteilhaft im Bereich von 60 bis 95 Gew.%, insbesondere 65 bis 85 Gew.%, bezogen auf die Gesamtmasse der Dispersion, gewählt.

Nachfolgende Beispiele erläutern die erfindungsgemäßen Hydrodispersionen. Die angeführten Zahlen sind, sofern nichts anderes angegeben, Gewichtsanteile bezogen auf die Gesamtmasse der Zubereitung.

### Beispiele:

| **INCI** | **F** | **G** | **H** | **I** |
|---|---|---|---|---|
| Ethylhexylglycerin | 0,5 | 0 | 0,5 | 0,15 |
| Caprylyl Glycol | 0 | 0,5 | 0 | 0 |
| Glycerin + Glyceryl Glucoside | 0,1 | 0,1 | 0 | 0,1 |
| Olus Oil | 0 | 0 | 3,2 | 0 |
| Candelilla Cera | 0 | 0 | 0,2 | 0 |
| Cetyl Palmitate | 3 | 3 | 1 | 0 |
| Paraffinum Liquidum | 0 | 0 | 0 | 3 |
| Butyrospermum Parkii Butter | 3 | 3 | 4 | 0,01 |
| Hydrogenated Vegetable Oil | 0 | 0 | 0,6 | 0 |
| C12-15 Alkyl Benzoate | 4 | 4 | 0 | 0 |
| Parfum | 0,2 | 0,2 | 0 | 0,25 |
| Glycerin | 10 | 10 | 9 | 1 |
| Aqua + Sodium Hydroxide | 1,09 | 1,09 | 0,2 | 0,1 |
| Shorea Stenoptera Seed Butter | 3 | 3 | 5 | 0 |
| Sodium Polyacrylate | 1,5 | 1,5 | 0,8 | 0 |
| Acrylates/C10-30 Alkyl Acrylate Crosspolymer | 0 | 0 | 0 | 0,2 |
| Aqua | 66,36 | 66,36 | 75,5 | ad 100 |
| Aqua + Trisodium EDTA | 1 | 1 | 0 | 0 |
| Phenylbenzimidazole Sulfonic Acid | 2,25 | 2,25 | 0 | 0 |
| Octocrylene | 2 | 2 | 0 | 0 |
| Butyl Methoxydibenzoylmethane | 2 | 2 | 0 | 0 |
| Methylisothiazolinone | 0 | 0 | 0 | 0,08 |
| Phenoxyethanol | 0 | 0,4 | 0 | 0,3 |

Die Hydrodispersionen zeigen sensorisch verbesserte Eigenschaften und deren Rückstand auf der Haut erscheint dem Anwender trotz Wachsgehaltes weniger wachsig.

Die kosmetischen und dermatologischen Hydrodispersionen zeigen ein angenehmes, sensorisch leichtes Profil und gute Pflegeeffekte und brechen bei Applikation auf der Haut nicht.

## Patentansprüche

1. Hydrodispersion umfassend Wasser, ein oder mehrere Verdickungsmittel, gegebenenfalls ein oder mehrere Wachse und gegebenenfalls ein oder mehrerer Öle **dadurch gekennzeichnet, dass** Ethylhexylglycerin und/oder Caprylylglycol enthalten ist.

2. Hydrodispersion nach Anspruch 1 umfassend 0,05 - 5 Gew.% wenigstens eines Verdickungsmittels, 2 bis 20 Gew.% eines oder mehrerer Wachse und bis 20 Gew.% eines oder mehrerer Öle, jeweils bezogen auf die Gesamtmasse der Dispersion.

3. Hydrodispersion nach einem der vorstehenden Ansprüche **dadurch gekennzeichnet, dass** die Dispersion keine anionischen Emulgatoren undTenside enthält.

4. Hydrodispersion nach einem der vorstehenden Ansprüche **dadurch gekennzeichnet, dass** die Dispersion keine kationischen Emulgatoren undTenside enthält.

5. Hydrodispersion nach einem der vorstehenden Ansprüche **dadurch gekennzeichnet, dass** das oder die Verdickungsmittel ausgewählt werden aus der Gruppe
• der Homopolymere oder Copolymere von Acrylsäure und/oder Acrylamid und deren Derivaten,
• der organischen, natürlichen Verbindungen, wie insbesondere Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Xanthan Gum,
• der organischen, abgewandelten Naturstoffe, wie insbesondere Carboxymethylcellulose, Hydroxyethyl- und/oder -propylcellulose, mikrokristalline Cellulose, und/oder
• der organischen, vollsynthetischen Verbindungen, wie insbesondere Polyacryl-und/oder Polymethacryl-Verbindungen, Vinylpolymere, Polycarbonsäuren, Polyether, Polyimine, Polyamide und/oder Polyurethane, AMPS Copolymere wie z.B. Ammonium Acryloyldimethyltaurate/VP Copolymer und Sodium Acrylate/ Acryloyldimethyltaurate/Dimethylacrylamide Crosspolymer, Acrylic Acid/VP Crosspolymer, PVP, Acrylamide/AmmoniumAcrylate Copolymer.

6. Hydrodispersion nach Anspruch 5 **dadurch gekennzeichnet, dass** als eines oder einziges der Verdickungsmittel Natriumpolyacrylat gewählt wird.

7. Hydrodispersion nach einem der vorstehenden Ansprüche **dadurch gekennzeichnet, dass** der Anteil an Verdickungsmitteln im Bereich 0,1 bis 2 Gew.%, bezogen auf die Gesamtmasse der Dispersion, gewählt wird.

8. Hydrodispersion nach einem der vorstehenden Ansprüche **dadurch gekennzeichnet, dass** der Anteil an Wasser gewählt wird im Bereich von 60 bis 95 Gew.%, insbesondere 65 bis 85 Gew.%, bezogen auf die Gesamtmasse der Dispersion.

9. Hydrodispersion nach einem der vorstehenden Ansprüche **dadurch gekennzeichnet, dass** der oder die Wachse gewählt werden aus der Gruppe der Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

10. Hydrodispersion nach Anspruch 9 **dadurch gekennzeichnet, dass** der oder die Wachse gewählt werden aus der Gruppe der Fette, insbesondere aus
• Shorea Stenoptera Seed Butter
• Hydrogenated Vegetable Oil
• Hydrogenated Coco-Glycerides
• Butyrospermum Parkii Butter
• Theobroma Cacao (Cocoa) Seed Butter
• Mango Butter,
aus der Gruppe der natürlichen Wachse, insbesondere aus
• Candellila Cera,
aus der Gruppe der Ester aus Fettsäuren, insbesondere aus
• Methyl Palmitate,
• Cetearyl Behenate,
• Cetyl Palmitate,
aus der Gruppe der synthetischen Wachse, insbesondere aus
• Synthetic Beeswax,
und/oder aus der Grupper der Fettalkohole, insbesondere aus
• Cetearyl Alcohol
• Cetyl Alcohol
• Stearyl Alcohol
• Behenyl Alcohol.

11. Hydrodispersion nach einem der vorstehenden Ansprüche umfassend 0,1 bis 1 Gew.% Ethylhexylglycerin und/oder Caprylylglycol, insbesondere im Bereich von 0,2 bis 0,6 Gew.%, bezogen auf die Gesamtmasse der Dispersion.

12. Hydrodispersion nach einem der vorstehenden Ansprüche umfassend weitere Konservierungsmittel und Konservierungsmittelhelfer zu einem Anteil von weniger als 1 Gew.%, insbesondere 0 Gew.%, bezogen auf die Gesamtmasse der Dispersion.

13. Kosmetische oder dermatologische Zubereitung umfassend oder bestehend aus einer Hydrodispersion nach einem der Ansprüche 1 bis 12.

14. Verwendung von Ethylhexylglycerin und/oder Caprylylglycol in Hydrodispersionen umfassend ein oder mehrere Verdickungsmittel, gegebenenfalls ein oder mehrere Wachse und gegebenenfalls ein oder mehrerer Öle zur Verdickung der Dispersion.

15. Verwendung von Ethylhexylglycerin und/oder Caprylylglycol in Hydrodispersionen, umfassend ein oder mehrere Verdickungsmittel, gegebenenfalls ein oder mehrere Wachse und gegebenenfalls ein oder mehrerer Öle, zur Konservierung der Dispersion ohne weitere Konservierungsmittel oder Konservierungsmittelhelfer.

16. Verwendung von Ethylhexylglycerin zur Verdickung von Hydrodispersionen im up-scaling Prozess.

17. Verwendung einer Hydrodsipersion nach einem der vorstehenden Ansprüche 1 bis 12 als Tränkungsmedium für Tücher mit einer Viskosität von mindestens 50mPas bis zu 1000 mPas.

18. Verwendung der Hydrodsipersion oder Zubereitung nach einem der Ansprüche 1 bis 13 als Hautpflegecreme.

19. Verwendung nach Anspruch 19 zur Anwendung am Auge.
